Europäisches Patentamt

European Patent Office

Office européen des brevets

(11) Publication number: **0 127 467 B1**

# EUROPEAN PATENT SPECIFICATION

(45) Date of publication of patent specification: **15.04.92**  (51) Int. Cl.⁵: **C07K 15/10, A01H 3/00**

(21) Application number: **84303558.5**

(22) Date of filing: **25.05.84**

(54) **Self incompatibility glycoprotein.**

(30) Priority: **27.05.83 AU 9578/83**

(43) Date of publication of application:
**05.12.84 Bulletin 84/49**

(45) Publication of the grant of the patent:
**15.04.92 Bulletin 92/16**

(84) Designated Contracting States:
**AT BE CH DE FR GB IT LI LU NL SE**

(56) References cited:

CHEMICAL ABSTRACTS, vol. 98, no. 15, 11th
April 1983, page 370, no. 122858e, Columbus,
Ohio, US; S. L. MAU et al.: " Isolation and
partial characterization of components of
Pruns avium L. styles, including an antigenic
glycoprotein associated with a self-
incompatibility genotype" & PLANTA 1982
(Pub. 1983). 156(6), 505-16

CHEMICAL ABSTRACTS, vol. 97, no. 15, 11th
October 1982, page 412, no. 124160z, Colum-
bus, Ohio, US; T. NISHIO et al.: "Comparative
studies on S-glycoproteins purified from dif-
ferent S-genotypes in self-incompatible
Brassica spices. I. Purification and chemical
properties" & GENETICS 1982, 100(4), 641-7

(73) Proprietor: **LUBRIZOL GENETICS INC.**
**29400 Lakeland Boulevard**
**Wickliffe Ohio 44092(US)**

(72) Inventor: **Clarke, Adrienne E.**
**35 Park Drive**
**Parkville Victoria 3052(AU)**
Inventor: **Mau, Shaio-Lim**
**2 Dalkeith Close**
**Wheelers Hill Victoria 3150(AU)**
Inventor: **Hoggart, Rosslyn**
**Lot 54, Cooloongatta Road**
**Sassafras Victoria 3787(AU)**
Inventor: **Anderson, Marilyn A.**
**47 Higgins Avenue**
**Sunbury Victoria 3429(AU)**
Inventor: **Williams, Elizabeth G.**
**24 Young Street**
**North Mitcham Victoria 3132(AU)**

(74) Representative: **Fisher, Adrian John**
**CARPMAELS & RANSFORD 43 Bloomsbury**
**Square**
**London WC1A 2RA(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 97, no. 21, 22nd November 1982, page 495, no. 178832p, Columbus, Ohio, US; K. HINATA et al.: "Comparative studies on S-glycoproteins purified from different s-genotypes in self-incompatible Brassica species. II. Immunological specificities" & GENETICS 1982, 100(4), 649-57

CHEMICAL ABSTRACTS, vol. 94, no. 25, 22nd June 1981, page 322, no. 205562x, Columbus, Ohio, US; G. M. M. BREDEMEIJER et al.: "S-specific proteins in styles of self-incompatible Nicotiana alata" & THEOR. APPL. GENET. 1981, 59(3), 185-90

BIOLOGICAL ABSTRACTS, vol. 63, 1st April 1977, no. 38504, Philadelphia, P.A., US; T.E. FERRARI et al.: "Pollen protein synthesis and control of incompatibility in Brassica" & THEOR APPL GENET 48(5): 243-249. 1976

BIOLOGICAL ABSTRACTS, vol. 66, 1st August 1978, no. 12547, Philadelphia, P.A., US; T. NISHIO et al.: "Stigma proteins in self-incompatible Brassica campestris I. and self-compatible relatives, with special reference to S-allele specificity" & JPN J GENET 53(1): 27-34. 1978

BIOLOGICAL ABSTRACTS, vol. 76, no.9, 1983, page 464, no. 68066, Philadelphia, P.A., US; E.G. WILLIAMS et al.: "The effect of isolated components of Prunus avium cultivar Napoleon styles on in vitro growth of pollen tabes" & PLANTA (BERL) 156(6):517-519. 1982

CHEMICAL ABSTRACTS, vol. 91, no. 17, 22nd October 1979, page 355, no. 137247j, Columbus, Ohio, US; I. N. ROBERTS et al.: "A glycoprotein associated with the acquistion of the self-incompatibility system by maturing stigmas of Brassica oleracea" & PLANTA 1979, 146(2), 179-83

## Description

This invention relates to the identification and isolation in substantially pure form of an antigenic protein material from mature styles of self-incompatible plants, particularly members of the Solanaceae and the Cruciferae, as exemplified by Nicotiana alata, an ornamental tobacco, and Lycopersicon peruvianum, a wild tomato, and Brassica oleracea, Brussel sprout. Studies of this style-specific material indicate that it corresponds to the self-incompatibility genotype of such self-incompatible plants, and is the product of the S-gene which controls self-incompatibility.

Accordingly, this material has potential for use in control of pollen-tube growth, for example, as a natural gametocide to control, induce, or promote self-incompatibility and interspecific incompatibility. The S-gene and its product can also be used in genetic manipulation of plants to create self-incompatible cultivars. Plants engineered in this way will be valuable for the economic production of hybrid seed. In summary, the utility of this material will be in (i) production of selfed seed in out-breeding crops; (ii) creation of new interspecific hybrid crops; and (iii) generation of methods for economic production of hybrid seed in currently self-compatible crops.

Many plant species, including Nicotiana alata and Lycopersicon peruvianum, are self-incompatible, that is, they cannot be fertilized by pollen from themselves or by that of a plant of the same S- (or self-incompatibility) genotype. The molecular basis of self-incompatibility is believed to arise from the presence of S-gene protein material in the mature styles of the plants; in particular, as exemplified by N. alata and L. peruvianum, this S-gene protein material has now been shown to be present in extracts of plant styles at the developmental stages of buds at first show of petal color, and at the subsequent stages of maturation of open but immature flowers, and flowers having mature glistening styles. On the other hand, the S-gene protein material is not present in the earlier developmental stages of green bud and elongated bud.

In plants exhibiting self-incompatibility, where the same S-gene is present in both the pollen and the pistil, pollen tube growth is arrested in the style of the plant and fertilization fails to occur. In both incompatible and compatible pollinations, the initial events are apparently identical. The pollen tubes germinate and grow extracellularly between the stigma papillae and then between the cells of the transmitting tissue of the style, which are joined by plasmodesmata into vertical files. At maturity, the extracellular material becomes progressively less viscous so that the pollen tubes grow through an amorphous fluid matrix between files of cells. At some point within the style, the growth of incompatible tubes is arrested. The precise zone of the style at which this happens varies with the ambient temperature. The behavior of the pollen tubes during growth indicates that the reaction may involve contact between the growing pollen tube and the extracellular components of the style, in particular contact of the growing tube with S-gene protein material as discussed above.

For general reviews of self-incompatibility, see de Nettancourt (1977), Incompatibility in Angiosperms, Springer-Verlag, Berlin; Heslop-Harrison (1978), Proc. Roy. Soc. London B, 202:73; Lewis (1979) N.Z. J. Bot. 17:637; Pandey (1979), N.Z. J. Bot. 17:645 and Mulcahy (1983) Science 220:1247. Self-incompatibility is defined as the inability of female hermaphrodite seed plants to produce zygotes after self-pollination. Two types of self-incompatibility, sporophytic and gametophytic, are recognized. In sporophytic incompatibility, pollen behavior is determined by the genotype of the pollen-producing plant. This type of incompatibility operates, for example in the Cruciferae and Compositae. If an allele of the S-gene in the pollen parent is also present in the female tissues, pollen tube growth is inhibited at the stigma surface. In gametophytic systems, pollen behavior is controlled by its own genotype. Growth of incompatible pollen tubes is arrested not at the stigma surface but within the style. Control is usually mediated through a single gene locus (S-gene) having multiple alleles. A pollen tube carrying a single given allele is inhibited if the same allele is present in the style. The gametophytic polyallelic system is considered to be the ancestral form of self-incompatibility in flowering plants, with the sporophytic system being derived from it. The products of the S-gene in the two systems may be structurally related.

There are five species of gametophytically self-incompatible plants and two species of sporophytically incompatible plants in which differences in style or stigma proteins apparently related to S-genotype have been detected electrophoretically or immunologically. In N. alata, an association between specific bands and three S-allele groups was demonstrated by isoelectric focussing of stylar extracts (Bredemeijer and Blaas (1981) Theor. Appl. Genet. 59:185). Two major antigenic components have been identified in mature styles of Prunus avium, one of which (S-antigen) was specific to a particular S-allele group (Raff, et al. (1981) Planta 153:125; and Mau, et al. (1982) Planta 156:505). The antigenic component, a glycoprotein, was isolated by gel-filtration and ion-exchange chromatography. Two dimensional sodium dodecylsulfate polyacrylamide gel electrophoresis (SDS-PAGE) resolved two components (molecular weights 37,000 and 39,000, respectively) which could correspond to the $S_3$ and $S_4$ products in the diploid stylar material of

cultivars of $S_3 S_4$ genotype. The material was a potent inhibitor of the in vitro growth of pollen tubes from a $S_3 S_4$ cultivar (Williams, et al. (1982) Planta 156:517). Stylar components which correspond to the S-allele group and are effective antigens in rabbits were detected in Petunia hybrida as reported by Linskens (1960), Z. Bot. 48:126. Four of the stylar proteins of Lilium longiflorum separated by electrofocussing reportedly showed distinct differences before and after heat treatment of the styles at 50°C. The S-gene product is thought to be heat labile because self-incompatibility can be broken by heat-treatment at 50°C; hence the bands lost during heat treatment may have corresponded to S-gene products (Dickinson, et al. (1982), Proc. Roy. Soc. London B. 215:45). A protein of molecular weight 24,000 isolated from stylar canal fluid of Trifolium pratense by Heslop-Harrison (1982) Ann. Bot. 49:729, may be involved in self-incompatibility.

A glycoprotein corresponding to genotypes $S_7$ of Brassica campestris has been isolated from extracts of stigmas by gel-filtration followed by affinity chromatography on Con-A-Sepharose (Trademark, Pharmacia, Inc. Uppsala, Sweden) then isoelectric focussing, Nishio and Hinata (1979) Jap. J. Genet. 54:307. The protein had a pI of 5.7, molecular weight (by SDS-PAGE) of 57,000 and contained protein and carbohydrate in the ratio 1:1.2. The protein was not present in styles or ovaries. Nishio and Hinata (1982) Genetics 100:641, used similar techniques to isolate S-specific glycoproteins from stigma extracts of Brassica oleracea plants homozygous for S-alleles $S_{39}$, $S_{22}$ and $S_7$. The glycoproteins had high pI's (10.3, 11.1 and 10.6 respectively) and contained protein and carbohydrate in the ratios of 1:0.05 ($S_7$ and $S_{22}$) and 1:0.2 ($S_{39}$). The apparent molecular weight (by SDS-PAGE) of glycoproteins $S_7$ and $S_{39}$ was 57,000. Glycoprotein $S_{22}$ was apparently heterogeneous; two dimensional SDS-PAGE resolved two components of molecular weights 60,000 and 65,000. Antisera raised to each isolated S-specific glycoprotein not only precipitated its homologous glycoprotein but also the other two S-specific glycoproteins of B. oleracea and the $S_7$-specific glycoprotein of B. campestris (Hinata et al (1982) Genetics 100:649). A similar S-specific glycoprotein was isolated by Ferrari, et al. (1981) Plant Physiol. 67:270, from a stigma extract of B. oleracea using different techniques. Pretreatment of $S_2 S_2$ pollen with the glycoprotein prevented the pollen germinating on normally compatible stigmas, indicating that the preparation was biologically active. Another partial purification was reported by Ferrari et al. (1981) using sucrose gradient sedimentation and double diffusion tests in gels in which the proteins were identified by Coomassie Blue staining.

Peroxidase isoenzymes extracted from styles of N. alata were reported to be S-gene specific by Pandey (1967), Nature 213:669. Subsequently, Bredemeijer and Blass (1980) Theor. Appl. Genet. 57:119, using the same species were unable to find any relationship between S-genotype and the peroxidase isozyme pattern and concluded that the peroxidases were coded by genes closely linked to the S-locus. It had been suggested that one of the isoenzymes in N. alata styles is involved in pollen tube growth (Bredemeijer and Blass (1975) Acta Bot. Naerl. 24:37), but it was subsequently found that this isoenzyme occurs in the cortex of the style, but is absent from the transmitting tissue, the site of pollen tube growth (Bredemeijer (1979) Acta Bot. Neerl. 28:197). Nasrallah, et al. (1970) Heredity 25:23, and Nishio and Hinata (1977) Heredity 38:391, were unable to detect any relationship between S-genotype and the pattern of peroxidase isoenzymes in stigma extract of B. oleracea. Therefore, the possible role, if any, of peroxidase isoenzymes in the incompatibility reaction is unclear.

In the present work, style extracts from the heterozygous self-incompatible gentoypes $S_1 S_3$ and $S_2 S_3$ of Nicotiana alata, as well as style extracts from the homozygous self-incompatible genotypes $S_2 S_2$ and $S_3 S_3$ generated from the heterozygous genotypes, have been examined for component materials corresponding to the self-incompatible genotype. Gel electrophoresis and immunological methods including immunoblot assays, have been used to investigate the properties of various stylar component materials. Similarly, style extracts from heterozygous self-incompatible genotypes $S_1 S_2$, $S_1 S_3$ and $S_2 S_3$ of L. peruvianum have been examined for S-protein materials.

As a result of this work, glycoprotein materials have been identified in the 30,000 MW region of stylar extracts of all genotypes $S_1 S_3$, $S_2 S_3$, $S_2 S_2$ and $S_3 S_3$, of N. alata and of genotypes $S_1 S_2$, $S_2 S_3$, $S_1 S_3$, $S_2 S_2$, $S_3 S_3$ and $S_3 S_4$ of L. peruvianum. These glycoprotein materials in the 30,000 MW region have been identified as the S-gene proteins as described hereinafter.

The term S-gene protein is used hereinafter to designate a protein coded by the S-gene. A protein coded by the S-gene must fulfill the following criteria: (1) segregation with S-allele; (2) localization in extracellular sites in the style. It should be in the mucilage of hollow styles or in the walls or intercellular matrix of the transmitting tract cells of styles with solid transmitting tissues. That is, the protein must be located at the site of contact with the pollen tubes; (3) Occurence of the protein in the developing style must be coincident with expression of self-incompatibility. In many species, self-incompatibility is not expressed in immature styles. The S-gene protein would not be expected to occur in such styles at these early developmental stages. In addition to the foregoing criteria, it will be understood that biological activity of the S-gene protein in an in vitro assay will provide corroboration that an S-gene protein, as defined above, is in itself functionally active for pollen tube growth inhibition. However, it is possible that the active component is a modified protein or a secondary product. In such cases, biological activity of an S-gene protein may require the activity of other components in order to be manifested in a bio-assay system. As used herein, the term protein includes glycoprotein, that is to say proteins modified to have one or more carbohydrate groups covalently bound thereto.

In the discussions of antibodies and antisera which follow hereinafter, unless otherwise specifically designated, the terms antibody and antiserum include both polyclonal and monoclonal antibodies and antisera.

Closely related, but distinct, glycoprotein materials are identified in SDS-PAGE gels which correspond to individual genotypes studied. For each genotype, the genotype-specific glycoprotein only appears as the flower matures, and is detected only in style extracts of buds at first show of petal color and in later stages of maturation, but not in the earlier bud stages. Two-dimensional gels confirm the genotype-specificity of these glycoproteins and indicate that they are characteristically of high isoelectric point, with the exception of L. peruvianum $S_1$-gene protein, which has a pI of 7.6. A significant aspect of the present invention is the discovery that rabbit antisera and monoclonal antibodies raised to stylar extracts show that all the genotype-specific glycoproteins are antigenic and that they cross-react immunologically, indicating considerable structural homology, if not complete identity, between the products of the different S-alleles. Immunological cross-reactivity between these S-genotype associated antigenic glycoproteins and extracts from styles of other self-incompatible crop and horticultural plants also indicates that the S-gene products in each case are highly conserved, that is they are closely related chemically.

Further details of these antigenic glycoprotein materials, and of their isolation and identification, are given in the following detailed description. In the accompanying drawings:

Figures 1 and 2 illustrate pollen tube growth in compatible and incompatible pollinations, respectively. Both figures are photomicrographs of sections of style cut lengthwise, and stained with a pollen tube-specific fluorescent stain after pollination. In Fig. 1, the style expressing both $S_3$ and $S_4$ alleles were pollinated by pollen expressing either $S_1$ of $S_2$ alleles. The pollen tubes grew directly to the ovary. In Fig. 2, the alleles of the pollen grains matched those in the style. Pollen growth was arrested in the upper part of the style.

Figure 3 shows the results of partial 2-dimensional gel electrophoresis of whole stylar extracts of mature L. peruvianum genotypes $S_1S_2$, $S_2S_3$ and $S_1S_3$, and of N. alata genotypes $S_2S_2$, $S_2S_3$, $S_3S_3$ and $S_1S_3$, with the bands associated with the various S-genotypes identified, as revealed by silver stain and by Coomassie Blue stain.

Figure 4 shows 2-dimensional gel electrophoretic separations of style extracts of L. peruvianum genotypes $S_1S_2$, $S_1S_3$, $S_2S_3$, $S_3S_3$, $S_3S_4$ and $S_2S_2$, with the bands associated with the various genotypes identified.

Figure 5 shows diagramatically the partial 2-dimensional gel electrophoresis procedure described herein.

Figure 6 shows the results of SDS-PAGE of whole stylar extracts of self-incompatible N. alata genotypes at various developmental stages.

Figures 7-10 show stages of the purification of the 32kd S-gene protein of N. alata, genotype $S_2$ on ion-exchange chromatography, followed by gel filtration or affinity banding and gel filtration.

Figure 12 shows the immuno-precipitation of N. alata style extracts of genotypes $S_2S_3$ and $S_3S_3$ with rabbit antisera raised against style extracts of L. peruvianum ($S_1S_3$), N. alata ($S_1S_3$), partially purified S-glycoprotein from Brassica campestris ($S_7$) and a purified S-glycoprotein from Prunus avium ($S_3S_4$).

Figure 13 shows immuno-cross-reactivity by immune precipitation of style extracts of N. alata, P. avium and L. peruvianum with antisera raised to partially purified S-genotype associated glycoprotein from Brassica campestris ($S_7$).

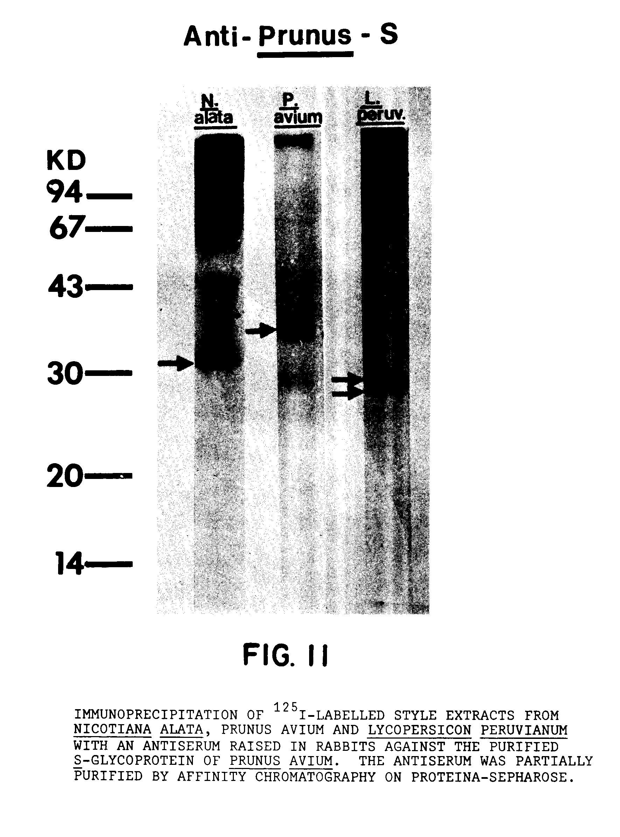

Figure 11 shows the immuno-precipitation of style extracts from N. alata, P. avium and L. peruvianum with purified rabbit antiserum against purified S-glycoprotein of P. avium.

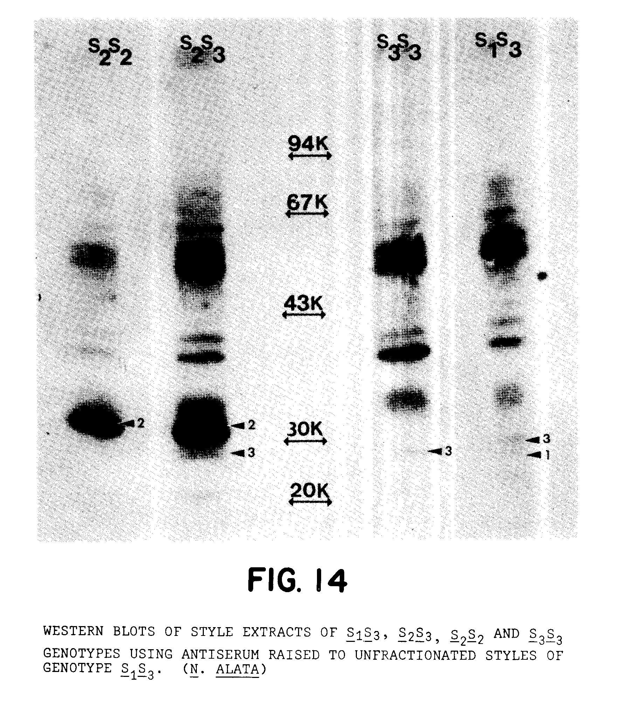

Figure 14 shows Western blots of style extracts of $S_1S_3$, $S_2S_3$, $S_2S_2$ and $S_3S_3$ genotypes using antiserum raised to unfractionated styles of genotype $S_1S_3$ (N. alata).

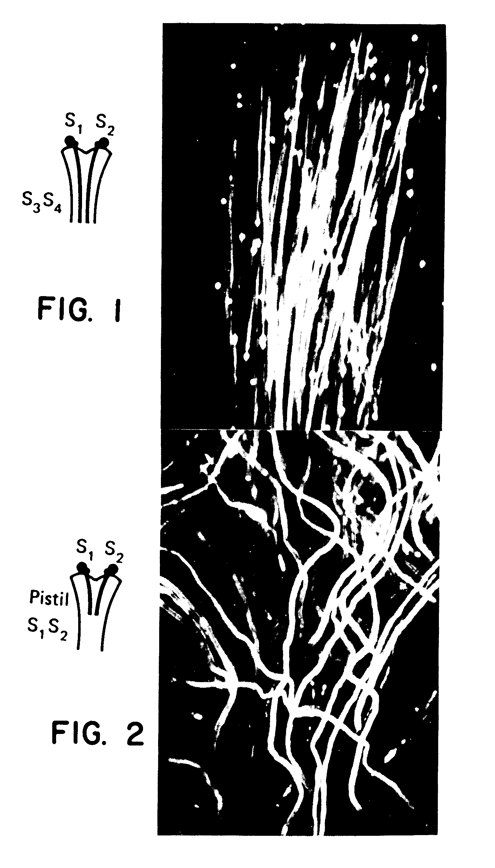

Referring firstly to Figures 1 and 2, there is shown in Figure 1 the normal growth of pollen tubes in an illustrative compatible pollination between, for example $S_1S_2$ and $S_3S_4$ genotypes, while Figure 2 shows the arrested pollen tube growth in a self-incompatible pollination between, for example, $S_1S_2$ genotypes.

The plant materials used in the present work were based on Nicotiana alata self-incompatible heterozygous genotypes $S_1S_3$ and $S_2S_3$ and on L. peruvianum heterozygous genotypes $S_1S_2$, $S_1S_3$, $S_2S_3$ and $S_3S_4$. Homozygous self-incompatibility genotypes were generated from these heterozygotes by self-pollination of flower buds since self-incompatibility is not expressed at early bud stage, but is expressed at the stage of development where green or pink color is showing on the bud. The method used was as follows:

Bud pollination to produce homozygotes

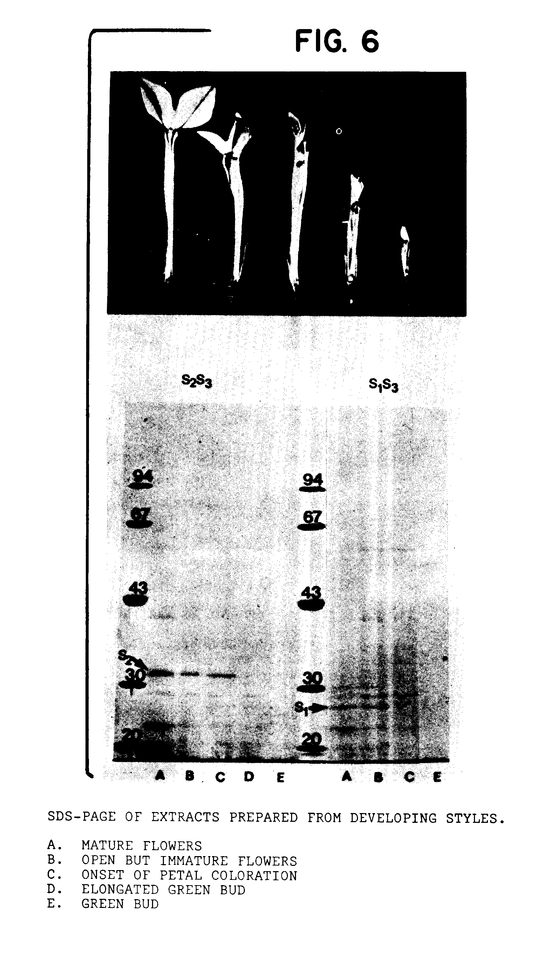

Buds generated from N. alata or L. peruvianum heterozygous plants $S_2 S_3$ and $S_1 S_3$ at the elongated green bud (stage D, Fig. 6) were emasculated by carefully slitting the corolla with fine forceps and gently removing the immature anthers. Twenty-four hours after emasculation (just prior to petal coloration, between stages D and C, Fig. 6) the immature stigma was pollinated with self pollen from a mature dehisced anther of another flower. Prior to pollination the stigma surface was coated with either:

(i) Exudate from a mature stigma applied by gently touching the two together or

(ii) 15% sucrose in 0.001% borate applied by carefully touching the stigma into a drop of this solution on a glass Petri dish.

After this treatment, the stigma was pollinated by gently touching it onto a glass Petri dish containing mature pollen or by brushing pollen carefully onto the stigma surface with a fine artist's brush. To prevent premature flower drop the flower axis was smeared with a little 1% (w/w) indole acetic (Sigma Cat. No. 1-1250) acid in raw lanoline using a flat ended spatula.

The bud-pollinated plants were kept in the Phytotron at 20°C and constant humidity and were labelled with key-tags placed on the floral axis. After six weeks the mature, dry seed pods were harvested.

The genotype of the $F_1$ progeny was established by test crossing against testers of known self-incompatibility genotype.

The general methods used in the isolation and identification of the antigenic glycoprotein materials are as follows:

Collection and storage of Styles: Mature, unpollinated styles were collected 24 to 48 hours after emasculation of buds at the stage of petal coloration (stage C, Fig. 6). Styles used for antisera production were either used immediately after collection or freeze dried and stored desiccated at -20°C. Styles extracted for protein analysis (gels or purification) were either fresh or stored at -70°C. (Style is used here to refer to the style plus stigma.)

Preparation of extract for raising specific antisera: Styles were ground, using a mortar and pestle, in 50mM Tris-HCl, pH 8.5 , 1mM $CaCl_2$, 1mM dithiothreitol (DTT), 10mM NaCl, 3% (w/v) polyvinyl pyrrolidone (PVP) at 0°C for 5 minutes. Approximately 400 styles (1g freeze-dried styles or 4.5g fresh styles) were used per 30ml extraction buffer. The extract was centrifuged at 10,000g for 15 minutes at 40°C: the clear green supernatant was removed and stored in 1ml aliquots at -20°C. Prior to injection of the rabbits, 1ml of the supernatant was passed down a Sephadex G-25 column (1cm x 20cm; Vo = 5.8ml) previously equilibriated with 0.005M sodium phosphate buffer, 0.15M sodium chloride, pH 7.4 (PBS).

Preparation of extract for gel electrophoresis and immunoblot assays: Styles were snap frozen in liquid nitrogen and ground to a fine powder using a mortar and pestle. This powder was extracted in 50mM Tris-HCl, pH 8.5, 1mM DTT, 10mM NaCl, 10mM EDTA, 1mM phenylmethylsulfonyl fluoride (PMSF), 1% (w/v) PVP at 0°C for 5 minutes. (100 styles per 10ml extraction buffer). The extract was centrifuged at 10,000g for 10 minutes at 4°C; the supernatant was removed and stored at -20°C in 1ml aliquots. Protein concentration of the extracts was determined as bovine serum albumin (BSA) using the Biorad protein assay kit (Biorad Laboratories, Inc., Richmond, California).

Preparation of antisera: Antisera to crude stylar extracts of N. alata were raised in rabbits by inoculation with 1mg of protein antigen in 0.5ml in Freund's complete adjuvant (1.5ml) administered either by multiple subcutaneous injections behind the neck or in a single dose intramuscularly in the flank. The rabbits were re-inoculated 6 weeks later with a further 1mg of antigen in Freund's incomplete adjuvant. After 7 to 10 days the rabbits were bled from the marginal ear vein, the sera collected and stored at -70°C in 200μl aliquots.

Immuno absorption of specific antisera: Immuno absorption was carried out by a single incubation of antiserum with freeze-dried styles (15 styles per 1ml antiserum) for 0.5, 1, 1.5, 2 or 2.5 hours at 20°C. The styles were removed and the antiserum centrifuged at 10,000g for 10 minutes at 4°C to remove any remaining debris.

Polyacrylamide gel electrophoresis: Sodium dodecyl sulphate polyacrylamide gel electrophoresis (SDS-PAGE) was performed according to Laemli, U.K., Favre, M. (1973) J. Mol. Biol. 80:575-583 using 12.5% (w/v) acrylamide. The samples were reduced by adding 1.43M 2-mercaptoethanol to the sample buffer and heating for 2 minutes in a boiling water bath. Molecular weight standards were phosphorylase B (94,000), bovine serum albumin (67,000), ovalbumin (43,000), carbonic anhydrase (30,000), soybean trypsin inhibitor (20,100) and β-lactalbumin (14,400) for Coomassie Blue staining and [14C]-methylated myosin (200,000), phosphorylase B (92,000), BSA (69,000), ovalbumin (46,000), carbonic anhydrase (30,000) and lysozyme (14,300) for electrophoretic blotting.

Electrophoresis was at 25mA per gel for the stacking gel and 40mA per gel for the running gel. After electrophoresis the gels were either stained with 0.14% (w/v) Coomassie Blue in 45% (v/v) methanol, 9% (v/v) acetic acid or were transferred to nitrocellulose paper.

Electrophoretic blotting: After electrophoretic separation of the proteins (20$\mu$g) in a polyacrylamide gel, the proteins were transferred and bound to nitrocellulose paper in an electrophoretic blot apparatus (Biorad Laboratories, Richmond, CA, USA) by electrophoresis at 0.1A for 12 hours, at 20°C in 20mM Tris-HCl, 20% methanol, 15mM glycine, pH 8.3.

Partial 2-dimensional gel electrophoresis

Style extracts of L. peruvianum were prepared by lightly crushing six styles with a fine glass rod in the presence of 20$\mu$l of extraction buffer (0.1M Tris-HCl pH8.5, 10mM EDTA, 0.1M NaCl, 1mM CaCl$_2$). The extract was centrifuged at 10,000g for 15 minutes at 4°C and the supernatant was collected and examined by two dimensional gel electrophoresis.

Extract (15$\mu$l containing approximately 15$\mu$g protein) was applied to an LKB Ampholine PAG plate, LKB-Produkter AB, Bromma, Sweden (24cm wide, 10cm long) containing ampholine (pH3.5-9.5). Isoelectric focussing was performed according to the Manufacturer's instructions with the following modifications: constant power (15 watts) at 10°C for 3.5 hours with 9cm between the electrodes. Electrode solutions were 1M NaOH (cathode) and 1M H$_3$PO$_4$ (anode). The gel was fixed with 10% TCA and 35% sulphosalicyclic acid for 30 minutes prior to staining with Coomassie Brilliant Blue.

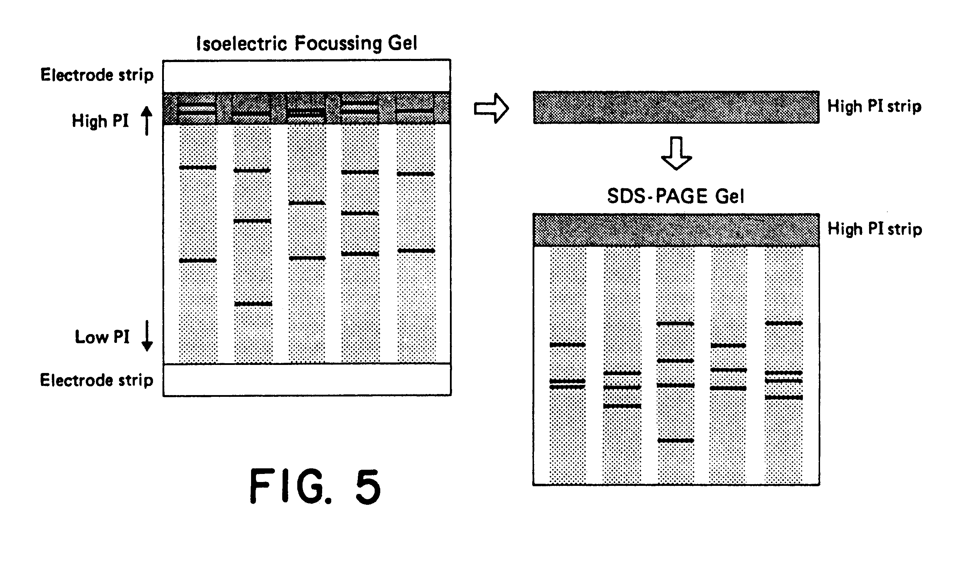

The protein track (approximately 0.5cm) or in some instances, proteins from a selected narrow pI range, from the isoelectric focussing gel was cut out and placed in 200ml of distilled water for 10 minutes, then transferred to 10ml of 1M Tris-HCl pH6.8 for 5 minutes. The gel strips were further rinsed with distilled water for 10 minutes before being equilibrated with SDS sample buffer for 1.5 hours at room temperature and placed on the top of an SDS-polyacrylamide gel as shown in Fig. 5. The second dimensional gel electrophoresis was carried out according to Laemmli et al. (1973) using 15% acrylamide. The protein was detected by staining with silver reagent or Coomassie blue.

Immuno-blot assays: After the proteins had been electrophoretically transferred to nitrocellulose paper, the remaining active sites on the paper were blocked by washing the paper in 3% (w/v) BSA in 10mM Tris-HCl, pH 7.4, 0.9% (w/v) NaCl, with gentle agitation for 1 hour at 20°C. Before addition of antiserum, the paper was rinsed in 10mN Tris-HCl, 0.9% (w/v) NaCl, pH 7.4 (Tris-saline) for 0.5 hour at 20°C. The antiserum was diluted 1 in 25, 1 in 100, or 1 in 200 with 3% (w/v) BSA in Tris-saline and incubated with the nitrocellulose paper for 1.5 hours at 20°C with gentle agitation. The nitrocellulose paper was washed for 10 minutes at 20°C in Tris-saline, followed by 10 minutes at 20°C in 0.05% (v/v) NP-40 (Trademark, Particle Data Labs. Elmhurst, Illinois) in Tris-saline, and then 10 minutes at 20°C in Tris-saline.

Protein A (40$\mu$g) was labelled with $^{125}$I; 0.3mCi using the Iodogen method (specific activity 1 x 10$^4$ to 1 x 10$^5$ cpm per $\mu$g Protein A). $^{125}$I-Protein A (20$\mu$g) was diluted to 200ml in 3% BSA in Tris-saline and incubated with the nitrocellulose paper for 0.5 hours with gentle agitation at 20°C. The nitrocellulose paper was washed in Tris-saline, then 0.05% NP-40 in Tris-saline for 10 minutes each at 20°C. The final wash was in Tris-saline containing 0.5% Triton X-100 (Trademark, Rohm and Haas Corp., Philadelphia, Pennsylvania), 0.1% SDS, 0.25% gelatine and 5mM EDTA for 1 hour at 20°C with gentle agitation. The paper was sealed in a plastic bag and exposed to Kodak XAR-5 x-ray film (Eastman Kodak Co., Rochester, NY), for 6-16 hours at - 70°C.

Immunoprecipitation conditions: 70$\mu$l of buffer (Tris-HCl, pH 7.4, 50mM; NaCl, 0.4M; EDTA, 5mM, KI, 5mM; NP-40, 0.05%; PMSF, 1mM), 20$\mu$l of iodinated extract (containing 1 --> 10 x 10$^6$ CPM), 5$\mu$l of ascites fluid or 10$\mu$l of non-immune mouse serum or 10$\mu$l of rabbit anti-style serum were mixed and incubated at 4°C for 1 hour. After this period, 10$\mu$l of anti-mouse IgG (anti-mouse IgG - Sigma M8890) was added and the mixture incubated for a further hour at 4°C. A suspension of Staph. aureus cells (20$\mu$l) was then added and the mixture centrifuged (10,000g; 5 minutes). The pellet was washed three times in immunoprecipitation buffer and then incubated at 90°C for 90 seconds with SDS-buffer (1 x Laemmli sample) and the resulting solution examined by SDS-PAGE using 12.5% buffer acrylamide.

Purification of 32K S$_2$-Glycoprotein from Nicotiana alata styles

Flowers from N. alata (genotype) S$_2$S$_2$) were emasculated at the onset of petal coloration. Two days later, the fully mature styles were removed and stored at -70°C. (Styles refer to the style and stigma which were removed together; ovary is not included.) Frozen styles (3g) were ground to a fine powder in liquid nitrogen using a mortar and pestle; this was followed by further grinding in 50ml of extracting buffer (50mm Tris-HCl, pH 8.5, 1mM $CaCl_2$, 10mM NaCl, 1mM DTT, 10mM EDTA and 1% (w/w) insoluble polyvinylpyrollidone. The homogenate was centrifuged (12,000g; 15 minutes) and the supernatant (11ml) was collected.

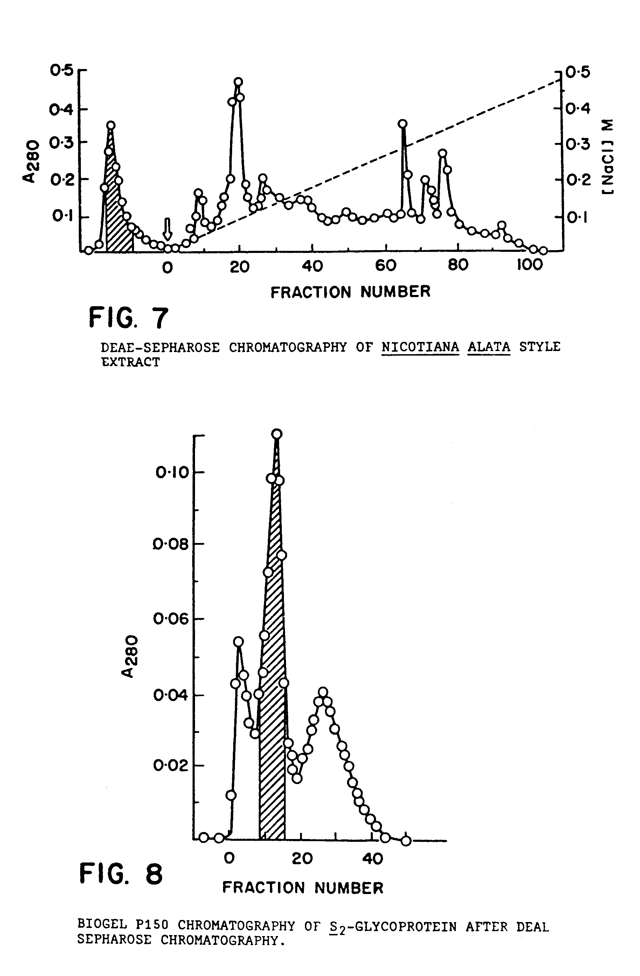

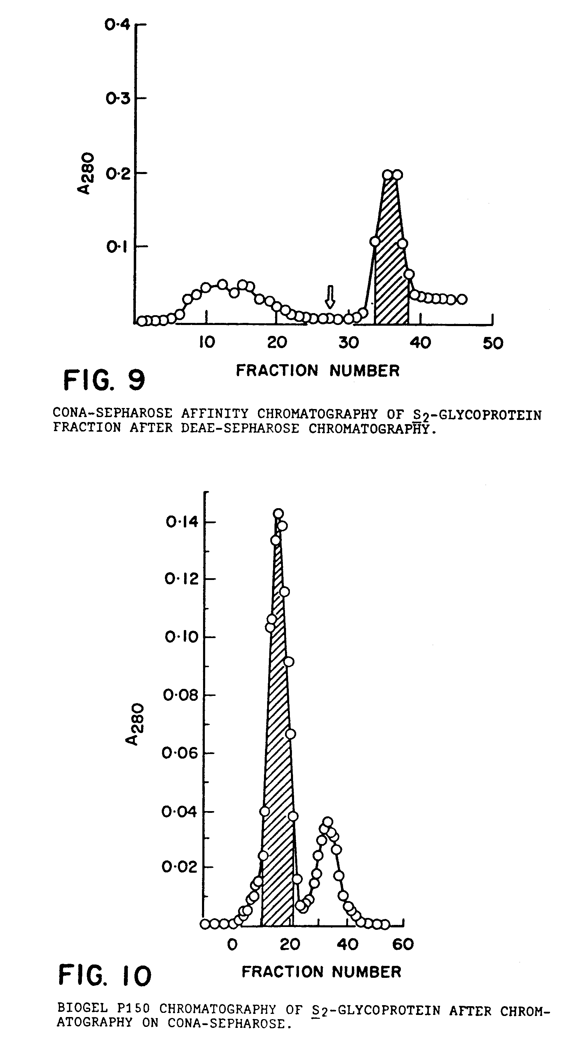

Prior to ion exchange chromatography the style extract (11ml) was equilibrated with $NH_4HCO_3$ 5mM pH 8.6, NaCl 1mM, $CaCl_2$, 1mM, EDTA 1mM by passage through a Sephadex G-25 (Trademark, Pharmacia Inc., Uppsala, Sweden) column (1.6cm diameter; 22cm long, void volume 11ml). The first 16ml eluted after the void volume was collected and applied to DEAE-Sepharose (Trademark, Pharmacia Inc., Uppsala, Sweden) (Bed volume 26ml, 1.6cm diameter x 13cm long) which was equilibrated with the same ammonium bicarbonate buffer. The column was then washed with this buffer (50ml) before the application of a NaCl gradient (0-0.5M). The elution profile is shown in Figure 7. Elution was monitored by $A_{280}$. Each fraction was examined by SDS-PAGE for the presence of the 32K $S_2$-glycoprotein. The $S_2$-glycoprotein was present in the unbound fractions (hatched area) which were combined as shown in Figure 7 and concentrated to a final volume of 16ml by rotary evaporation at room temperature. The $S_2$-glycoprotein was further purified by size fractionation on Biogel P150 (Trademark, BioRad Laboratories, Richmond, California) (Figure 8) or by affinity chromatography using ConA-Sepharose (Trademark, Pharmacia Inc., Uppsala, Sweden) followed by gel filtrations on 3:0 gel P-150 (Figures 9 and 10).

The unbound fraction from DEAE-Sepharose (8ml) was applied to a column of Biogel P150 (void volume 14ml; 1.6cm in diameter, 36.5cm long) which was equilibrated with $NH_4HCO_3$ 10mM, pH 8.5, EDTA 10mM, NaCl 0.1M, $CaCl_2$ 1mM. The elution profile is shown in Figure 8. Fractions were examined for the presence of the 32K $S_2$-glycoprotein by SDS-PAGE. The fractions containing the $S_2$-glycoprotein (Fractions 9-16) were combined and concentrated to 1ml by rotary evaporation.

ConA-Sepharose was washed with 5 volumes of methyl-$\alpha$-D-mannoside (0.1M) in buffer (sodium acetate 20mM, pH 7.8; NaCl 0.1M, $MgCl_2$ 1mM, $CaCl_2$ 1mM, $MnCl_2$ 1mM). The washed ConA-Sepharose was then transferred to bicarbonate buffer (0.25M $NaHCO_3$ pH 8.8) for 1 hour at room temperature; the bicarbonate buffer was changed 4 times during the 1 hour period. Four volumes of $NaHCO_3$ 0.25M, pH 8.8 containing 0.03% (v/v) glutaraldehyde were added and the ConA-Sepharose was then washed with $NaHCO_3$ 0.1M, pH 8.0, containing 0.5M NaCl, resuspended in acetate buffer (sodium acetate 10mM, pH 7.8; NaCl 0.1M, $MgCl_2$ 1mM; $CaCl_2$ 1mM, $MnCl_2$ 1mM and packed into a column (0.8cm diameter, 14cm long). The unbound fraction from DEAE-Sepharose was equilibrated in acetate buffer, by passing through a G25-Sepharose column equilibrated with acetate buffer, then applied to the column. Unbound material was collected, the column washed with 10 volumes of acetate buffer, and the bound material eluted with 0.1M methyl-$\alpha$-D-mannoside in acetate buffer. The profile is shown in Figure 9. Fractions 34-38 were collected and concentrated to 1ml by rotary evaporation.

The pooled fraction eluted by 0.1M methyl-$\alpha$-D-mannoside was applied to a column of Biogel P150 to separate the methyl-$\alpha$-D-mannoside from the $S_2$-glycoprotein (Figure 10) (void volume 13ml, 1.6cm diameter; 36.5cm long equilibrated and run in $NH_4HCO_3$ 10mM pH 8.5, EDTA 10mM, NaCl 0.1M $CaCl_2$ 1mM). A further passage through Biogel P2 in water, to remove any trace of methyl-$\alpha$-D-mannoside, was used before undertaking carbohydrate analysis of the $S_2$-glycoprotein.

## Recovery of protein applied

|  | mg protein | % recovery |
|---|---|---|
| Style extract recovered from Sephadex G-25 chromatography | 28.6 |  |
| Fraction not bound to DEAE-Sepharose | 2.79 | 9.7 |
| Biogel P150 (Fractions 9-16) | 0.56 | 2.0 ⎫ |
| ConA-Sepharose (Fractions 34-38) | 0.49 | 1.7 ⎬ 3.7 |

The purified S-gene protein was essentially homogenous by the criteria of SDS-PAGE and two-dimensional gel electrophoresis using either silver stain or Coomassie Blue stain to visualize the bands. N-terminal sequence analysis showed that the purified material has less than 5% contamination with other proteins. The foregoing purification scheme is applicable, with such modifications as may be deemed appropriate to those of ordinary skill in the art, to the purification of others S-gene proteins, from other plant

8

sources. The purification scheme yields S-gene protein in substantially purified form. As used herein, the term "substantially purified form" means that the purified material migrates as a single component on SDS-PAGE, when stained either with silver stain or with Coomassie Blue stain, and N-terminal sequencing shows less than 5% contamination with other proteins.

RESULTS

Partial 2-dimensional gel electrophoresis of whole stylar extracts

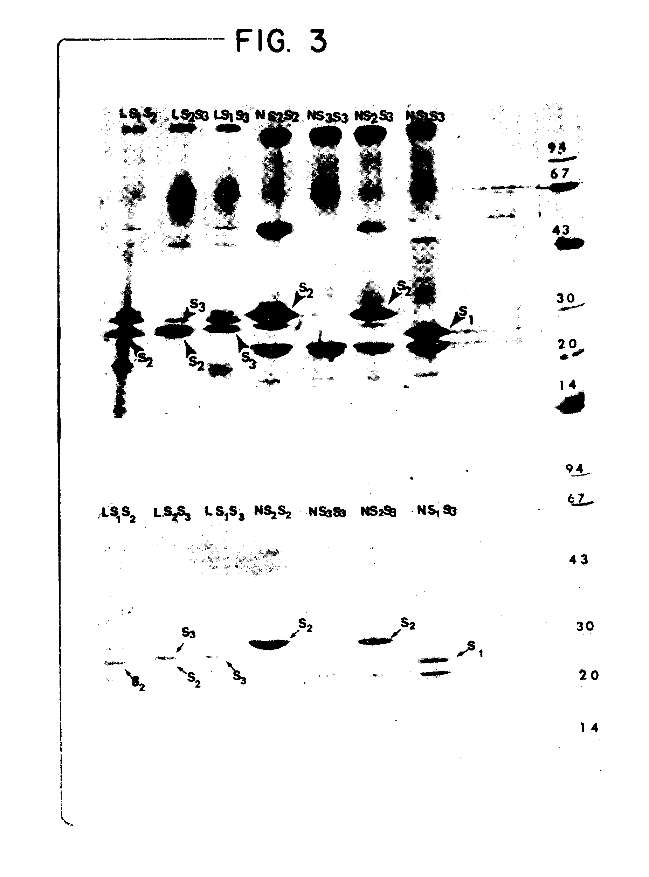

N. alata $S_1S_3$, $S_2S_3$, $S_2S_2$, and $S_3S_3$, and L. peruvianum $S_1S_2$, $S_2S_3$, $S_1S_3$, $S_2S_2$ and $S_3S_3$ showed a complex pattern of proteins in the MW range 20K to 120K. Most bands are common to all genotypes, but there are differences in the 20K-30K region which can be associated with S-genotype. These differences are apparent in the gels after staining with silver stain (Fig. 3, upper part) and with Coomassie blue (Fig. 3, lower part). Each lane in Fig. 3 is designated by genus initial (L = Lycopersicon, N = Nicotiana) and self-incompatibility genotype. Molecular weight markers are shown on the extreme right of each gel set.

The molecular weights of the S-gene proteins associated with each N. alata genotype are approximately 27 kilodaltons (K) for $S_1$, and 32K for $S_2$, as determined with molecular weight markers. The $S_3$-gene product appeared to migrate at about 23K; however the simultaneous presence of a common band has made unequivocal designation difficult. S-gene specific proteins in the 23K-35K molecular weight range were consistently observed to be characteristic of their corresponding S-allele and to fit all the criteria described herein for S-gene proteins. Purified N. alata $S_2$-gene protein migrated as a single component on both SDS-PAGE and two-dimensional gels, as revealed either by Coomassie Blue or by silver stain. L. peruvianum styles yielded genotype-specific protein bands with molecular weights of approximately 25K-28K for each of $S_1$, $S_2$ and $S_3$-gene proteins. The proteins were further shown to be S-gene specific by 2-dimensional gel electrophoresis and by other criteria, as disclosed, infra.

2-dimensional gel electrophoresis of whole stylar extracts of mature L. peruvianum genotype

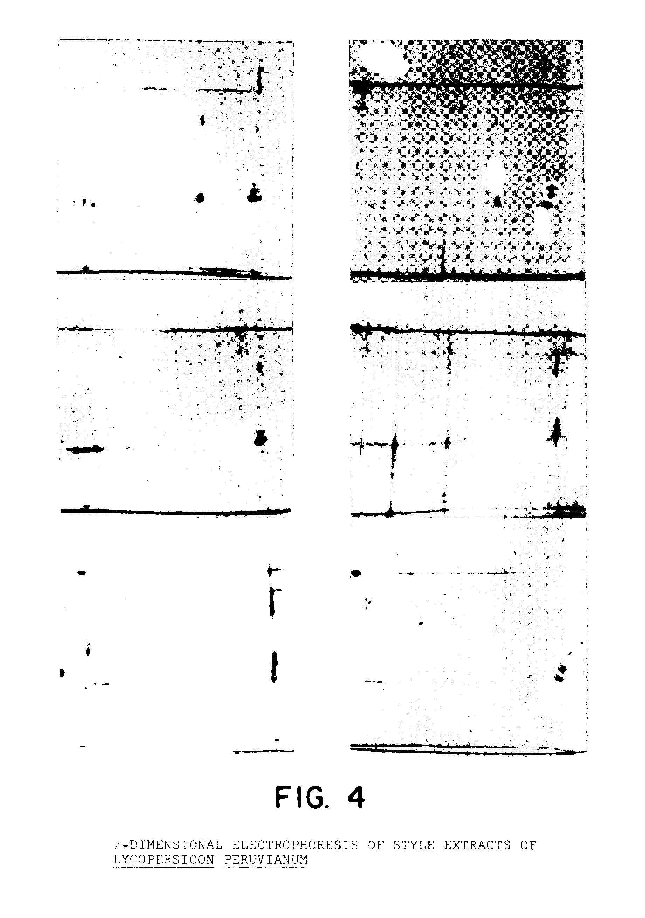

Distinct proteins were similarly observed in style extracts of $S_1S_3$, $S_1S_2$, $S_2S_3$, $S_3S_3$, $S_3S_4$ and $S_2S_2$ - (Fig. 4). Molecular weights of $S_1$-, $S_2$- and $S_3$-gene proteins were all approximately 28K. $S_1$-gene protein had a lower pI than did $S_2$, $S_3$ and $S_4$. Two-dimensional gel electrophoresis was performed essentially as described by O'Farrell, et al. (1977) Cell 12:1133. The proteins fall into a major group of pI > 7.5 and a minor group of low pI. The S-gene-proteins were found in the high pI group. In addition to the genotype-associated bands, designated in Fig. 4, there were two distinct bands at pI > 9.5 common to all style extracts, running close to the genotype associated bands but distinguished from them by their higher molecular weight. Other bands in the lower pI range were found to differ from one extract to another, but no association of these bands with a particular genotype could be assigned.

Stylar proteins associated with style maturation

Extracts of N. alata $S_1S_3$ and $S_2S_3$ styles from the developmental stages of green bud, elongated bud, buds at first show of petal color, open but immature flowers and mature flowers with glistening styles, were prepared and subjected to SDS-PAGE (Fig. 6 - this figure also illustrates the various developmental stages). A number of proteins were detected in all extracts of which those tentatively identified as the S-gene proteins in the 30K MW region, were present in extracts of buds at first show of petal color and in subsequent stages of maturation, but not in the earlier bud stages. The appearance of the bands tentatively assigned to S-genotype is summarized in Table 1. Similar results were observed for the S-gene proteins of L. peruvianum. In one experiment, $S_2$ and $S_3$ gene proteins were present only in very low concentrations in extracts of green buds. There was a progressive increase in the apparent concentration of these components in extracts of styles taken from yellow buds and mature flowers. These results further confirm the identity of the described proteins as S-gene proteins according to the criteria set forth herein.

9

TABLE 1

Appearance of proteins corresponding to $\underline{S}$-genotype in extracts of <u>Nicotiana alata</u> ($\underline{S_1S_3}$ and $\underline{S_2S_3}$) styles at different stages of maturity.

| | Presence of putative $\underline{S}$-gene product | | | |
|---|---|---|---|---|
| Developmental Stage | Genotype $\underline{S_1S_3}$ | | Genotype $\underline{S_2S_3}$ | |
| | $\underline{S_1}$ | $\underline{S_3}$ | $\underline{S_2}$ | $\underline{S_3}$ |
| Δ early bud | - | - | - | - |
| Δ elongated bud | - | - | - | - |
| * buds at first show of petal color | + | + | + | + |
| * open, immature flowers | + | + | + | + |
| * mature, flowers | + | + | + | + |

Δ Incompatibility not expressed - bud self-pollinations are successful.

* Incompatibility expressed - self-pollinations not successful.

<u>Further studies demonstrating identity of N. alata 32K protein as $S_2$-gene protein</u>

The 32K glycoprotein is present in the stigma and in the upper style sections in higher concentrations than in the lower sections of the style, as revealed by SDS-PAGE of extracts of 5mm style sections. The highest concentration of the 32K glycoprotein was found in the upper part of the style, which is also the zone in which pollen tube inhibition occurs. Similarly, in L. peruvianum, the $S_2$ and $S_3$ gene proteins were present in relatively high concentrations in the first segment containing the stigma and the top of the style and the next style segment (2mm segments). The apparent concentration of these components decreased progressively in segments of the style taken towards the ovary, and none were detected in the ovary itself.

No bands corresponding to the N. alata 32K band were found in style extracts of N. tabaccum, N. silvestris, N. glauca, or Petunia hybrida on SDS-PAGE. No bands corresponding to the 32K style band was found in extracts of other N. alata tissues, including petal, leaf, ovary and anther. The 32K band is therefore not a protein common to other species or to other N. alata tissues.

Biological activity of 32K $S_2$-gene protein was demonstrated in the in vitro pollen tube growth assay described by Williams, et al. (1982). $S_2$-gene protein at $25\mu$g/ml inhibited the growth of pollen tubes by about 70% provided the extraction buffer did not contain EDTA or other divalent ion binding agent. The results demonstrated that a biologically effective concentration of $\underline{S}$-gene protein can act to inhibit pollen tube growth.

While the foregoing experiments have been carried out in greatest detail using the $\underline{S_2}$-gene protein of

N. alata and $S_2$ and $S_3$ of L. peruvianum it will be apparent to those ordinarily skilled in the art that the similarities in timing of developmental expression, physical properties, association with self-incompatibility genotype and immunological properties taken together demonstrate that other S-gene proteins as described herein have been identified from a variety of plant species. As a result of the findings and teachings herein disclosed, S-gene proteins of self-incompatible species can now be identified, isolated, purified and characterized for use in plant breeding. A significant property of S-gene proteins, demonstrating their fundamental structural similarity across species boundaries, is next demonstrated.

Immunological cross-reactivity of S-gene protein from different plants

An antiserum was raised in rabbits against the S-protein of Prunus avium, purified as described by Mau, et al. (1982). Style extracts from N. alata, P. avium and L. peruvianum were labeled with $^{125}$I by the Iodogen technique of Fraker and Speck (1978) Biochem. Biophys. Res. Comm. 80:849. Labeled extracts were immunoprecipitated following the procedure detailed, supra. The anti-P. avium S-protein serum precipitated the 32K $S_2$-protein of N. alata and the S-proteins of approximately 28K of L. peruvianum (which revealed two bands since the source plant carried two S-alleles) in addition to P. avium S-protein (37K), as shown in Fig. 11.

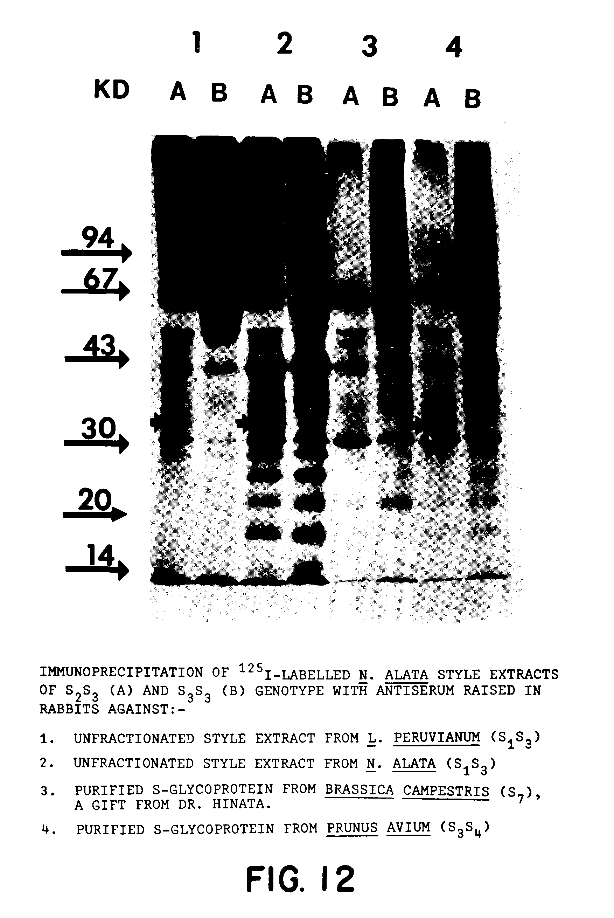

Using essentially the same technique, a variety of antisera were shown to immuno-precipitate $^{125}$I-labeled N. alata style extracts of genotypes $S_2S_3$ and $S_3S_3$. The results are shown in Fig. 12. Lanes A and B in each pair represent genotypes $S_2S_3$(A) and $S_3S_3$(B). Each numbered lane pair shows immunoprecipitation with a rabbit antiserum against: 1) L. peruvianum $S_1S_3$ unfractionated style extract; 2) N. alata $S_1S_3$ unfractionated style extract; 3) Brassica campestris $S_7$ partially purified S-glycoprotein [prepared and provided by K. Hinata, see Nishio and Hinata (1982)]; 4) P. avium $S_3S_4$ purified S-glycoprotein. The positions of molecular weight markers are shown on the left margin. Arrows show the position of 32K $S_2$-protein of N. alata. The $S_2$- glycoprotein was immunoprecipitated by heterologous antisera in each instance with the apparent exception of $S_2$ treated with anti-Brassica serum. However, as shown in Fig. 12, N. alata $S_2$-protein was precipitable by anti-Brassica serum.

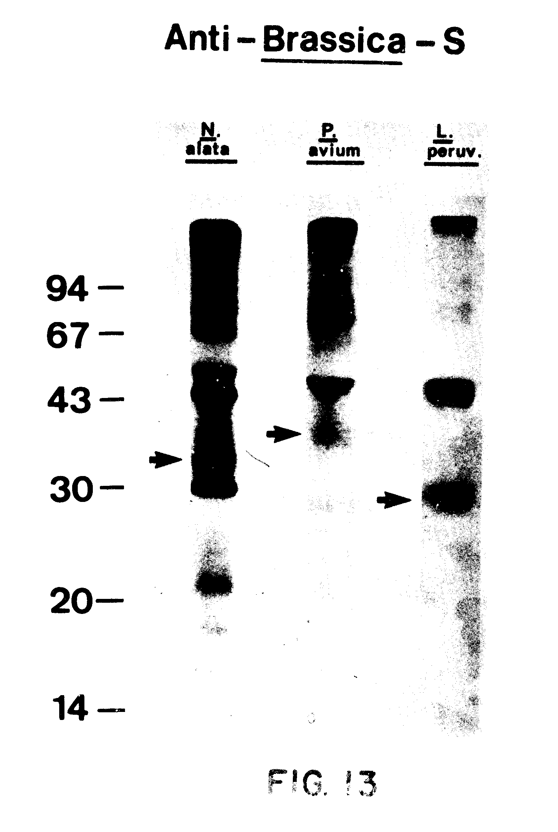

In Fig. 13, the results of immunoprecipitation analysis using antiserum raised to Brassica campestris partially purified S-protein (provided by K. Hinata) are shown. The serum was able to immunoprecipitate S-protein in style extracts from N. alata, P. avium, and L. peruvianum, as marked by the arrows in the respective lanes of Fig. 13. Molecular weight marker positions in Fig. 13 are shown in the left hand margin.

Antigenic properties of stylar extracts

Antiserum to both N. alata $S_1S_3$ and $S_2S_3$ whole-stylar extract bound to proteins of stylar extracts of four N. alata genotypes in a Western blot analysis, the only detectable differences in binding patterns occurring in the 30K MW region. The pattern of antiserum binding is such that an association of a particular band with S-genotype can be assigned (Fig. 14). Normal rabbit serum did not bind to any of the extracts examined. A number of high MW proteins also reacted with the antiserum, but these were common to style extracts of all genotypes.

The fact of immunological cross-reactivity between S-proteins of different genotype within the same species, between S-proteins of different species and even between species having gametophytic incompatibility (Nicotiana, Lycopersicon) and sporophytic incompatibility (Brassica) indicates that these S-proteins have at least a single common component with homologous structures. Despite apparent differences in molecular weight and pI, the evidence disclosed herein reveals the surprising fact that S-gene proteins are a recognizable structural class, in addition to having functional similarities.

Uses of S-gene proteins and antibodies to these proteins

The availability of S-gene proteins and antibodies against them makes a variety of new plant breeding strategies possible. The difficulty of producing selfed seed in self-incompatible, outbreeding crops makes it very difficult to develop and maintain homozygous elite lines. The difficulty can be overcome by the use of an anti S-protein serum, which, when applied to the stigma with appropriate timing, can render a normally self-incompatible plant temporarily self-compatible. Conversely, a normally self-compatible plant can be rendered temporarily self-incompatible, and thereby prevent self-fertilization, by using S-protein applied by suitable means, for example as a spray or coating on the stigma surface. (A self-incompatible plant will normally reject pollen of a self-fertile (Sf) plant).

New interspecific hybrids can be created by techniques that exploit S-gene proteins. For example, domestic tomato cultivars can be improved by genetic inputs, such as pathogen resistance, drought tolerance and the like, from related wild species. A self-fertile cultivar, used as the female parent, is rendered temporarily self-incompatible by application of an S-gene protein, preferably that of a close, self-incompatible relative, to the stigmas of the plant. Such a phenotypic modification is symbolized by $\underline{S}_f\underline{S}_f(\underline{S}_1)$. Pollination by a self-incompatible parent bearing a desired trait, designated by * will result in F1 progeny carrying one self-incompatibility allele and the desired trait.

$$\underline{S}_f\underline{S}_f(\underline{S}_1) \times \underline{S}_2\underline{S}_2{}^* \text{---} \tfrac{1}{2} \underline{S}_f\underline{S}_2{}^*$$

Such F1 progeny cannot self-pollinate and will not set seed unless treated to inactivate the S-protein, for example, by use of anti-$S_2$ serum. However, such F1 plants may be themselves useful if they are of a crop where only vegetative parts are harvested, e.g., Brassica. Furthermore, the F1 plants can be used as male parents for backcrossing to the original $S_fS_f$ parent. By recurrent backcrosses to the $S_fS_f$ parent, accompanied by selection for the * gene, an $S_fS_f{}^*$ cultivar carrying that gene can be developed.

The foregoing strategy can be extended to produce hybrid seed. In one such scheme, four parental varieties are employed:

| Variety 1 | Variety 2 | Variety 3 | Variety 4 |
|---|---|---|---|
| $\underline{S}_f\underline{S}_f$ | $\underline{S}_2\underline{S}_2{}^*$ | $\underline{S}_f\underline{S}_f$ | $\underline{S}_1\underline{S}_1{}^*$ |

Varieties 1 and 3 are rendered temporarily self-incompatible by treatment with an appropriate S-gene protein. For example, for a cross between Var. 1 and Var. 2, Var. 1 is treated with an $S_1$-gene protein, to render it self-incompatible but compatible with Var. 2. It will be understood that Var. 1 and Var. 3 may in fact be identical, if desired, although they will be rendered temporarily distinct phenotypically by the S-gene protein treatment. Varieties 2 and 4 may be naturally self-incompatible. However, in certain instances it will be preferred to introduce the self-incompatibility alleles especially for the purpose, using any available and appropriate genetic manipulation. In crosses

<center>Var. 1 x Var. 2                     Var. 3 x Var. 4</center>

$$\underline{S}_f\underline{S}_f(\underline{S}_1) \times \underline{S}_2\underline{S}_2{}^* \qquad \text{and} \qquad \underline{S}_f\underline{S}_f(\underline{S}_2) \times \underline{S}_1\underline{S}_1{}^*$$

the F1 progeny will be $S_fS_2{}^*$ and $S_fS_1{}^*$, respectively. As before, such plants are self-incompatible. However, the two F1 types are cross-compatible and a mixture of seed of both types will yield plants which can fertilize each other and set seed to produce a crop. ($1S_fS_1 : 2S_1S_2 : 1S_fS_2$)

The S-gene proteins and antibodies thereto are also of use to plant breeders to determine the self-incompatibility alleles carried by a given plant cultivar. Heretofore, such determinations required laborious and time-consuming crossing experiments. In vitro pollen tube growth assays can be used in combination with purified S-gene proteins of each known S-allele to identify the S-genotype of a pollen sample. Alternatively, specific S-gene protein antibodies can be used to identify corresponding proteins in style extracts. Antibodies that are allele-specific are preferred for distinguishing various allelic S-gene proteins from one another. For a given plant species the test materials can be produced in the form of a kit comprising a set of S-gene proteins, plates for in vitro pollen tube growth and buffered media for promoting pollen tube growth. A test kit for measuring S-gene proteins immunochemically, either quantitatively or qualitatively, would comprise a set of antibodies appropriate to the plant species, together with means for identifying the binding of an S-gene protein with its antibody. Such means could include fluorescent-dye coupled antibody, or enzyme-conjugated antibody to permit detection by fluorescence of an antigen-antibody complex, or by activity of the antibody-enzyme conjugate, the latter assay system being generally known in the art as enzyme-linked immunoabsorbent assay (ELISA). Other immunoassay detection means may be used, as appropriate, in accordance with principles of operation well known to those of ordinary skill in the art. For field testing and routine lab analysis, an ELISA is preferred.

Those skilled in the art will appreciate that the invention described herein and the methods of isolation and identification specifically described are susceptible to variations and modifications other than as specifically described. It is to be understood that the invention includes all such variations and modifications which fall within its spirit and scope.

## EP 0 127 467 B1

### Claims

1. An S-gene glycoprotein having a molecular weight of between 23 kd and 35 kd, said glycoprotein being obtained from a plant style of a gametophytically self-incompatible plant which is a member of the family Solanaceae, said glycoprotein segregating with a single self-incompatibility allele of said plant and having the ability to inhibit pollen tube growth in said plant only from pollen having the same self-incompatibility allele.

2. A glycoprotein of claim 1 having the ability to immunologically cross-react with antibody to any of the S1-gene proteins of Nicotiana alata, the S2-gene protein of Nicotiana alata, the S3-gene protein of Nicotiana alata, the S1-gene protein of Lycopersicon peruvianum, the S2-gene protein of Lycopersicon peruvianum, the S3-gene protein of Lycopersicon peruvianum, the S7-gene protein of Brassica campestris, campestris, or S-gene of Prunus avium.

3. A glycoprotein of claim 1 wherein the gametophytically self-incompatible plant is a member of the genus Nicotiana.

4. A glycoprotein of claim 1 wherein the gametophytically self-incompatible plant is a member of the genus Lycopersicon.

5. A glycoprotein of claim 1 selected from proteins comprising the self-incompatibility alleles S1, S2, and S3 of Nicotiana alata and S1, S2 and S3 of Lycopersicon peruvianum.

6. An antiserum prepared from the glycoprotein of any preceding claim.

7. A method of preparing a glycoprotein according to any of claims 1 to 5 in substantially pure form, comprising the steps of
    (a) extracting frozen ground mature styles with an aqueous buffer,
    (b) fractionating the extract by ion exchange chromatography on an anion exchanger eluted with a salt gradient,
    (c) fractionating S-gene protein-containing fractions from step (b) by affinity chromatography on ConA cross-linked to a chromatographic support matrix and eluted with an α-glycoside in aqueous solution,
    (d) pooling S-gene protein containing chromatographic fractions derived from step (c), whereby said glycoprotein is obtained in substantially pure form.

### Revendications

1. Une glycoprotéine de gène S ayant un poids moléculaire compris entre 23 kd et 35 kd, ladite glycoprotéine étant obtenue à partir d'un style de plante, d'une plante gamétophytiquement auto-incompatible qui fait partie de la famille des Solanacées, ladite glycoprotéine se ségrégant avec un seul allèle d'auto-incompatibilité de ladite plante et ayant la capacité d'inhiber la croissance du tube pollénique dans ladite plante seulement vis-à-vis de pollen ayant le même allèle d'auto-incompatibilité.

2. Une glycoprotéine de la revendication 1, ayant la capacité de réaction croisée immunoloqiquement avec un anti-corps vis-à-vis de toutes les protéines de gène S1 de Nicotinia alata, la protéine de gêne S2 de Nicotinia alata, la protéine de gène S3 de Nicotinia alata, la protéine de gène S1 de Lycopersicon peruvianum, la protéine de gène S2 Lycopersicon peruvianum, la protéine de gène S3 de Lyopersicon peruvianum, la protéine de gène S7 de Brassica campestris ou le gène S de Prunus avium.

3. Une glycoprotéine de la revendication 1 dans laquelle la plante gamétophytiquement auto-incompatible fait partie du genre Nicotiana.

4. Une glycoprotéine de la revendication 1 dans laquelle la plante gamétophytiquement auto-incompatible est un membre du genre Lycopersicon.

5. Une glycoprotéine de la revendication 1, choisie parmi les protéines comprenant les allèles S1, S2 et

13

S3 d'auto-incompatibilité de Nicotinia alate et S1, S2 et S3 de Lycopersicon peruvianum.

6. Un antisérum préparé à partir de la glycoprotéine de l'une quelconque des revendications précédentes.

7. Un procédé pour préparer une glycoprotéine selon l'une quelconque des revendications 1 à 5 sous forme sensiblement pure, comprenant les phases consistant à
(a) extraire les styles matures broyés, gelés au moyen d'un tampon aqueux,
(b) fractionner l'extrait par chromatographie à échange d'ions sur un échangeur d'anions élué au moyen d'un gradient de sel,
(c) fractionner les fractions contenant la protéine du gène S de la phase (b) par chromatographie à affinité sur un ConA à liaison croisée avec une matrice de support chromatographique et éluée au moyen d'un α-glycoside en solution aqueuse,
(d) rassembler la protéine du gène S contenant des fractions chromatographiques dérivées de la phase (c) grâce à quoi ladite glycoprotéine est obtenue sous forme sensiblement pure.

**Patentansprüche**

1. S-Gen-Glykoprotein mit einem Molekulargewicht zwischen 23 kd und 35 kd, das aus einem Pflanzengriffel einer gametophytisch selbstinkompatiblen Pflanze aus der Familie der Solanaceae gewonnen wird, welches Glykoprotein sich mit einer einzigen Selbstinkompatibilitätsallele dieser Pflanze absondert und die Fähigkeit hat, das Pollenschlauchwachstum in dieser Pflanze nur von jenem Pollen zu inhibieren, der dieselbe Selbstinkompatibilitätsallele hat.

2. Glykoprotein nach Anspruch 1 mit der Fähigkeit zur immunologischen Kreuzreaktion mit einem Antikörper gegen irgendeines der S1-Gen-Proteine von Nicotiana alata, das S2-Gen-Protein von Nicotiana alata, das S3-Gen-Protein von Nicotiana alata, das S1-Gen-Protein von Lycopersicon peruvianum, das S2-Gen-Protein von Lycopersicon peruvianum, das S3-Gen-Protein von Lycopersicon peruvianum, das S7-Gen-Protein von Brassica campestris oder das S-Gen von Prunus avium.

3. Glykoprotein nach Anspruch 1, wobei die gametophytisch selbstinkompatible Pflanze ein Mitglied der Gattung Nicotiana ist.

4. Glykoprotein nach Anspruch 1, wobei die gametophytisch selbstinkompatible Pflanze ein Mitglied der Gattung Lycopersicon ist.

5. Glykoprotein nach Anspruch 1, ausgewählt aus den Proteinen, die die Selbstinkompatibilitätsallelen S1, S2 und S3 von Nicotiana alata und S1, S2 und S3 von Lycopersicon peruvianum umfassen.

6. Antiserum, hergestellt aus dem Glykoprotein nach irgendeinem der vorhergehenden Ansprüche.

7. Verfahren zur Herstellung eines Glykoproteins nach einem der Ansprüche 1 bis 5 in praktisch reiner Form, umfassend folgende Verfahrensschritte:
(a) Extraktion von reifen, in gefrorenem Zustand gemahlenen Griffeln mit einem wässerigen Puffer,
(b) Fraktionierung des Extrakts durch Ionenaustauschchromatographie auf einem Anionentauscher, der mit einem Salzgradienten eluiert wird,
(c) Fraktionierung der S-Gen-Protein-haltigen Fraktionen aus Stufe (b) durch Affinitätschromatographie auf ConA, das an eine chromatographische Trägermatrix gebunden und mit einem alpha-Glykosid in wässeriger Lösung eluiert ist, sowie
(d) Vereinigen der S-Gen-Protein-haltigen chromatographischen Fraktionen aus Stufe (c), wodurch dieses Glykoprotein in praktisch reiner Form erhalten wird.

FIG. 1

FIG. 2

# FIG. 3

# FIG. 4

2-DIMENSIONAL ELECTROPHORESIS OF STYLE EXTRACTS OF
LYCOPERSICON PERUVIANUM

FIG. 5

EP 0 127 467 B1

# FIG. 6

SDS-PAGE OF EXTRACTS PREPARED FROM DEVELOPING STYLES.

A. MATURE FLOWERS
B. OPEN BUT IMMATURE FLOWERS
C. ONSET OF PETAL COLORATION
D. ELONGATED GREEN BUD
E. GREEN BUD

## FIG. 7

DEAE-SEPHAROSE CHROMATOGRAPHY OF NICOTIANA ALATA STYLE EXTRACT

## FIG. 8

BIOGEL P150 CHROMATOGRAPHY OF $S_2$-GLYCOPROTEIN AFTER DEAL SEPHAROSE CHROMATOGRAPHY.

FIG. 9

CONA-SEPHAROSE AFFINITY CHROMATOGRAPHY OF $S_2$-GLYCOPROTEIN
FRACTION AFTER DEAE-SEPHAROSE CHROMATOGRAPHY.

FIG. 10

BIOGEL P150 CHROMATOGRAPHY OF $S_2$-GLYCOPROTEIN AFTER CHROM-
ATOGRAPHY ON CONA-SEPHAROSE.

**Anti- Prunus - S**

FIG. II

IMMUNOPRECIPITATION OF $^{125}$I-LABELLED STYLE EXTRACTS FROM
NICOTIANA ALATA, PRUNUS AVIUM AND LYCOPERSICON PERUVIANUM
WITH AN ANTISERUM RAISED IN RABBITS AGAINST THE PURIFIED
S-GLYCOPROTEIN OF PRUNUS AVIUM.  THE ANTISERUM WAS PARTIALLY
PURIFIED BY AFFINITY CHROMATOGRAPHY ON PROTEINA-SEPHAROSE.

IMMUNOPRECIPITATION OF $^{125}$I-LABELLED N. ALATA STYLE EXTRACTS OF $S_2S_3$ (A) AND $S_3S_3$ (B) GENOTYPE WITH ANTISERUM RAISED IN RABBITS AGAINST:-

1. UNFRACTIONATED STYLE EXTRACT FROM L. PERUVIANUM ($S_1S_3$)

2. UNFRACTIONATED STYLE EXTRACT FROM N. ALATA ($S_1S_3$)

3. PURIFIED S-GLYCOPROTEIN FROM BRASSICA CAMPESTRIS ($S_7$), A GIFT FROM DR. HINATA.

4. PURIFIED S-GLYCOPROTEIN FROM PRUNUS AVIUM ($S_3S_4$)

FIG. 12

# Anti – Brassica – S

FIG. 13

# FIG. 14

WESTERN BLOTS OF STYLE EXTRACTS OF $S_1S_3$, $S_2S_3$, $S_2S_2$ AND $S_3S_3$ GENOTYPES USING ANTISERUM RAISED TO UNFRACTIONATED STYLES OF GENOTYPE $S_1S_3$. (N. ALATA)